# NEUE EUROPÄISCHE PATENTSCHRIFT

(11) **EP 1 154 969 B2**
(45) Veröffentlichungstag und Bekanntmachung des Hinweises auf die Entscheidung über den Einspruch: **29.06.2011**
(45) Hinweis auf die Patenterteilung: 14.05.2003
(21) Anmeldenummer: 00909147.1
(22) Anmeldetag: 04.02.2000
(51) Int. Cl.: C04B 35/64, A61K 6/06, C04B 35/111, C04B 35/486

(54) **VERFAHREN ZUM DIMENSIONSTREUEN SINTERN VON KERAMIK**
METHOD FOR SINTERING DIMENSIONALLY PRECISE CERAMICS
PROCEDE POUR LE FRITTAGE, ADAPTE AUX DIMENSIONS, DE CERAMIQUE

(30) Priorität: 04.02.1999 DE 19904523
(43) Veröffentlichungstag der Anmeldung: 21.11.2001
(73) Patentinhaber: 3M ESPE AG, 82229 Seefeld (DE)
(72) Erfinder: HAUPTMANN, Holger, D-82404 Sindelsdorf (DE); BURGER, Bernd, D-82239 Alling (DE); SCHNAGL, Robert, D-86899 Landsberg (DE); WAGNER, Ingo, D-82237 Wörthsee (DE)
(86) Internationale Anmeldenummer: PCT/EP2000/000909
(87) Internationale Veröffentlichungsnummer: WO 2000/046166

(56) Entgegenhaltungen:
- EP-A- 0 530 370
- EP-A- 0 583 620
- EP-A1- 0 583 620
- WO-A2-96/29951
- DD-A1- 121 025
- US-A- 3 733 171
- US.Z: ROCK, MISIOLEK: 'Distortion Control for P/M-Based Rapid Prorotyping of Advance Material Components.' 16 Juni 1996 - 21 Juni 1996, WORLD CONGRESS ON POWDER METALLURGY & PARTICULATE MATERIALS, Seiten 1 - 15
- US.Z.: AMAYA: 'Size & Distortion Control in MIM Processing' ADVANCES IN POWDER METALURGY Bd. 2, Seiten 285 - 295
- 'Katalog 97/98', Artikel DE.Z.: RENFERT, Seite 60
- 'Modern Practice in Dental Ceramics', 1967, W.B. SAUNDERS COMPANY, PHILADELPHIA & LONDON Seiten 280-285, - 290,291
- 'VMK 95, Bilder', Juli 1998 Artikel VITA ZAHNFABRIK H.RAUTER GMBH & CO.KG,
- 'Keramik heute', Dezember 1986 Artikel VITA ZAHNFABRIK H. RAUTER GMBH & CO.KG, Seiten 8 - 9

## Beschreibung

Die Erfindung betrifft ein Verfahren zum dimensionstreuen Sintern von freiformflächigen Keramiken. Insbesondere betrifft die Erfindung ein Verfahren zum dirnensionstreuen Sintem von aus Dentalkeramiken hergestellten Dentalprothesen.

Keramiken werden aufgrund ihrer physikalischen Eigenschaften bei der Erstellung von hochwertigen Formteilen, beispielsweise Zahnersatzteilen, sehr geschätzt und finden daher immer breitere Verwendung. Beim Sintem von keramischen Werkstoffen tritt stets eine Volumenreduzierung (Schwund) ein. Teile des zu sintemden Objektes führen während des Brennvorganges eine Relativbewegung zu einer starren, nicht beweglichen Brennunterlage aus. Bei filigranen Arbeiten, die insbesondere im Bereich des Zahnersatzes eingesetzt werden, wird die freie Beweglichkeit durch geringfügige Verhakungseffekte auf der Brennunterlage behindert, wodurch eine erhebliche Deformation des Objektes auftritt. Besonders kritisch ist dieser Sachverhalt bei Brücken, die beispielsweise aus zwei Käppchen und einem diese verbindenden Steg bestehen: es tritt eine Deformation der ursprünglichen Geometrie der Brücke auf, die die Passgenauigkeit der prothetischen Arbeit erheblich beeinträchtigt.

Üblicherweise werden Pulver zur Reduzierung der Reibung zwischen Brenngut und Brennunterlage verwendet. Bei höheren Sintertemperaturen treten jedoch entweder Reaktionen zwischen Pulver und Brenngut oder ein Verbacken der Pulverschüttung durch Ausbildung von Sinterhälsen auf. In beiden Fällen kann dies zu dem oben beschriebenen Effekt führen und somit zur Unbrauchbarkeit des Brennguts. Durch das Eigengewicht der Rohlinge bedingt, kann es bei Systemen, die Superelastizität aufweisen, zusätzlich zur Verformung der Rohlingsstrukturen kommen. Insbesondere tritt dieser Effekt bei Brücken auf.

Aus der DD-121 025 ist es bekannt, Formlinge auf Brennunterlagen zu brennen, die mit Molybdän beschichtet sind. Solche Verfahren sind prinzipiell für hochwertige keramische Werkstücke ungeeignet, da durch Diffusionsprozesse eine Verunreinigung der Keramik durch Metallteilchen erfolgt.

Aus der EP 0 530 370 A1 ist ein Verfahren zur Herstellung eines Oxidsuperleiters bekannt, bei welchem ein pulverförmiges Material, das zu einem Oxidsuperleiter geformt werden soll, auf Silber oder Silberoxid in einer Pfanne aufgebracht wird, die Pfanne auf eine Temperatur oberhalb der Schmelztemperatur des Silbers erhitzt wird, um das genannte Material in einen halbgeschmolzenen Zustand zu überführen, während es auf dem geschmolzenen Silber schwimmt, und die Pfanne abgekühlt wird und das Material dann von dem sich wieder verfestigten Silber abgetrennt wird, wodurch ein rißfreies Material mit einem Durchmesser von 10 cm oder mehr erhalten werden kann.

Die EP 0 583 620 A1 beschreibt ein Verfahren zum Sintern von Siliciumnitridgegenständen in Schüttungen aus verschiedenen Pulvern.

Die WO 96/29951 beschreibt ein Verfahren zur Herstellung von zahnprothetischen Rekonstruktionen, wobei die dreidimensionale Kontur der Kavität bzw. des Zahnstumpfes oder der Form des Abutments bestimmt und eine um einen vorgegebenen Vergrößerungsfaktor vergrößerte, dreidimensionale Form des Zahninlays bzw. der Zahnkrone oder des Abutments aus vorbehandeltem Zahnersatz-Material erzeugt wird, welche Form nachbehandelt wird, wobei eine Schrumpfung auf ein der Kavität bzw. dem Zahnstumpf oder Abutments entsprechendes Maß erfolgt, wobei ein bestimmter Arbeitsstumpf und/oder eine Arbeitspackung aus einem Material erzeugt wird, das in etwa den gleichen Schrumpffaktor wie das Zahnersatz-Material aufweist, der Arbeitsstumpf zur Nachbehandlung in die Form eingebracht und/oder die Arbeitspackung zur Nachbehandlung von außen um die Form gepackt wird, wodurch die Form während der Nachbehandlung stabilisiert wird und der Arbeitsstumpf bzw. die Arbeitspackung nach erfolgter Nachbehandlung von der Form getrennt wird.

Aufgabe dieser Erfindung ist es, ein Verfahren zur Verfügung zu stellen, das ein dimensionstreues Sintern von keramischen Formgegenständen erlaubt.

Erfindungsgemäß wird diese Aufgabe gelöst durch Verfahren, wie sie in den Ansprüchen beschrieben sind.

Die erfindungsgemäßen Träger können vollkommen verschieden ausgestaltet sein. Die Ausgestaltungsformen sind prinzipiell in folgende Gruppen zu unterteilen:
I Lagerung des Brennguts auf beweglichen Trägern, die aus einem beliebigen Material bestehen können, beispielsweise basierend auf gesintertem Aluminiumoxid, welches gegenüber dem Brennprozess inert ist und keine Haftung zum Brenngut ergibt und dieses nicht verunreinigt.
II Lagerung des Brennguts auf Trägern die die gleichen physikalischen Eigenschaften aufweisen wie das Brenngut selbst. Der Träger besteht hierbei aus dem gleichen Material, wie das Brenngut, beispielsweise basierend auf Zirkonoxid oder Aluminiumoxid.

Mögliche Ausführungsformen zur Gruppe I der erfindungsgemäßen Verfahren sind nachfolgend wiedergegeben.

Prinzipiell wird bei dieser Verfahrensvariante das Brenngut auf einem beweglichen Träger gelagert. Diese Träger sind in einem Fundament zu lagern oder über eine Aufhängung zu befestigen.

Als Fundament sind insbesondere folgende Ausführungsformen geeignet:
- Feuerfeste Brennwatte, beispielsweise ein Vlies aus Aluminiumoxid mit SiO₂-Anteil.
- Feuerfester Brennsand, beispielsweise Korund.
- Nach oben offene, unterteilte Konstruktionen, beispielsweise wabenförmige Konstruktionen, in denen ein Verkippen der beweglichen Träger im Rahmen des Brennprozesses auf einfache Weise möglich ist, beispielsweise solche aus Mullit.
- Feuerfeste Einbettmassen, die eine genügende Flexibilität aufweisen, um den Kräften, die während des Brennvorgangs auftreten, auszuweichen, beispielsweise solche aus Aluminiumoxid.
- Feuerfeste, den gleichen Schwund wie das Brenngut aufweisende Grundplatten, beispielsweise solche aus Aluminiumoxid.

Als Aufhängung sind insbesondere folgende Ausführungsformen geeignet:
- Aufhängung über fest montierte Haken, wobei das Brenngut an geeigneter Position auf mindestens zwei Haken aus feuerfestem Material, beispielsweise Aluminiumoxid, aufgezogen wird, und die Haken sich durch die während des Brennprozesses auftretenden Kräfte annähern.

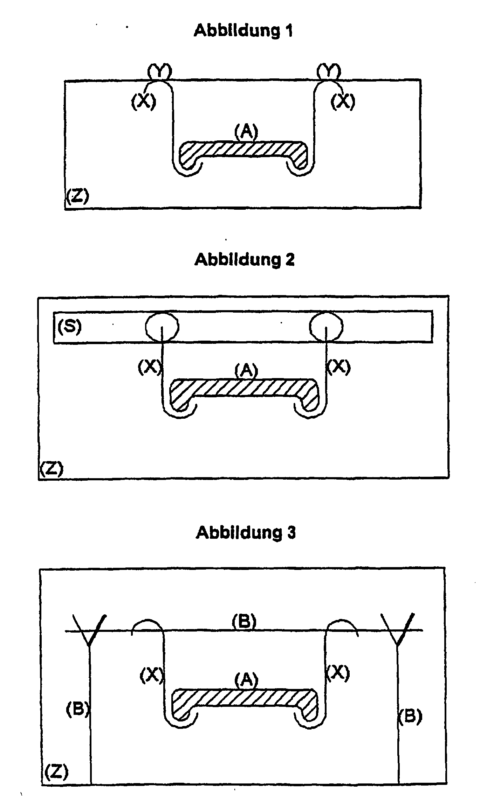

   Die Abbildung 1 zeigt exemplarisch die Anbringung zweier S-förmiger Haken (X) an einer festen Stelle (Y) innerhalb einer Brennkammer (Z), wobei das Brenngut (A) bereits auf die Haken aufgezogen ist. Die Ausgestaltung des Brenngutes ist hier und an allen anderen Stellen nur schematisch wiedergegeben und ist keinesfalls beschränkend zu verstehen.
- Aufhängung über beweglich angebrachte Haken, wobei das Brenngut an geeigneter Position auf mindestens zwei Haken aus feuerfestem Material, beispielsweise aus Aluminiumoxid, aufgezogen wird und die Haken innerhalb oder außerhalb der Brennkammer beweglich angebracht sind.
   Die Abbildung 2 zeigt exemplarisch die Anbringung zweier S-förmiger Haken (X) innerhalb der Brennkammer (Z), wobei die Haken jeweils auf einer Schiene (S), beispielsweise über Rollen, frei beweglich sind und so den Kräften, die während dem Brennprozess entstehen, ausweichen können und das Brenngut (A) bereits auf die Haken aufgezogen ist.
   Die Haken können auch in eine barrenförmigen Schienenkonstruktion (B) eingehängt werden, wie sie in der Abbildung 3 gezeigt wird. Die Konstruktion besteht aus senkrechten Elementen von (B) und waagrechten Elementen von (B), die eine Aufhängung der Haken (X), die das Brenngut (A) tragen, ermöglichen.
   Prinzipiell kann jede Methode, mindestens zwei Haken flexibel in einer geeigneten Höhe zu befestigen, Anwendung finden.

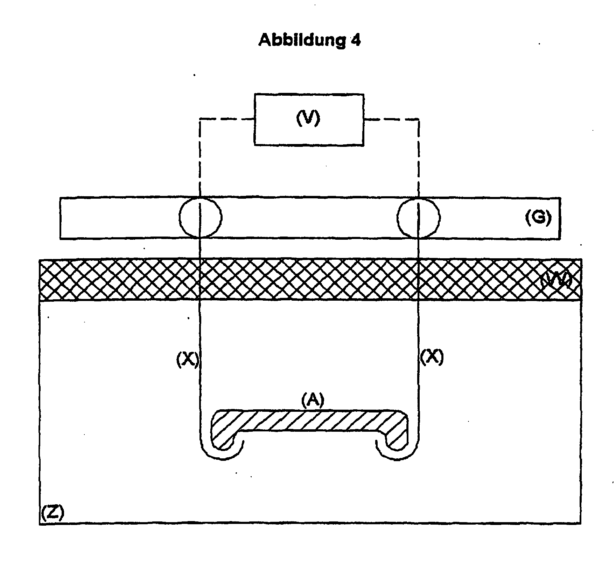

   Die Abbildung 4 zeigt exemplarisch die Anbringung zweier Haken (X) außerhalb der Brennkammer (Z), wobei die Haken jeweils auf einem Gleitlager (G) frei beweglich sind und so den Kräften, die während dem Brennprozess entstehen, ausweichen können. Da sich die beweglichen Träger außerhalb der Brennkammer befinden, wird das Verfahren vorzugsweise so angewandt, dass die Brennkammer über eine geeignete Wärmeisolation (W) von den Trägem abgeschirmt ist. Diese Variante des erfindungsgemäßen Verfahrens kann auch dadurch verbessert werden, dass die Bewegung der Haken in den Gleitlagern nicht ausschließlich durch die während des Brennprozesses auftretenden Kräfte stattfindet, sondern dass durch eine mechanische, elektronische und/oder optische Abtastvorrichtung (V) die für einen Kraftausgleich notwendige Positionsänderung der Haken in den Gleitlagern ermittelt und beispielsweise mechanisch ausgeführt wird (Prinzip des Tangentialplattenspielers).
- Als Aufhängung im Sinne dieser Erfindung werden auch Vorrichtungen verstanden, die das gleiche Prinzip, wie zuvor beschrieben, verwenden, die Gleitlager jedoch unterhalb des Brenngutes angebracht sind, wobei diese sich innerhalb oder außerhalb der Brennkammer befinden können.

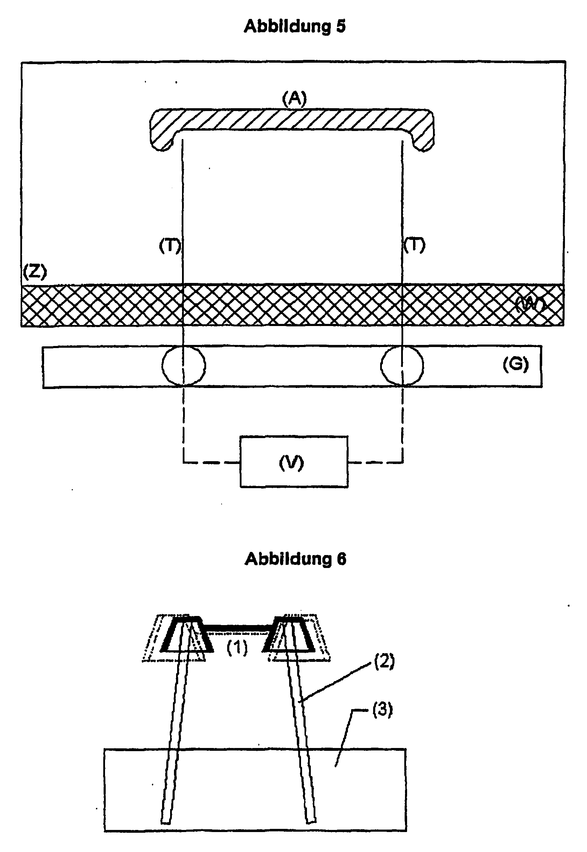

   Die Abbildung 5 zeigt exemplarisch die Anbringung zweier Stützen (T) für das Brenngut, wobei die Stützen auf Gleitlager (G) außerhalb der Brennkammer (Z) frei beweglich sind und so den Kräften, die während des Brennprozesses entstehen, ausweichen können. Eine Wärmeisolation (W) kann hier ebenso wie eine mechanische, elektronische und/oder optische Abtastvorrichtung (V), die die für einen Kraftausgleich notwendige Positionsänderung der Haken in den Gleitlagern ermittelt und beispielsweise mechanisch ausführt, vorteilhaft sein.

Als Träger bzw. Stützen sind insbesondere folgende Ausführungsformen geeignet:
- Stäbchen, die einen Querschnitt aufweisen, der eine minimale Berührungsfläche mit dem Brenngut gestattet, beispielsweise kreisförmige, ellipsenförmige, rechteckige, insbesondere quadratische und rautenförmige, konvexe, konkave, dreieckige, U-förmige Querschnitte, wobei die Stäbchen hohl oder massiv sein können; die Stäbchen können dabei senkrecht stehend oder waagrecht liegend angeordnet sein.
- Trägern, die eine Spitze aufweisen, die eine minimale Berührungsfläche mit dem Brenngut gestattet, beispielsweise pfeilförmige, pyramidenförmige, kegelförmige Träger, die hohl oder massiv sein können.

Die benannten Träger, Rollen, Aufhängungen oder Stützen können aus allen refraktären Metallen, Metalloxiden, Metallcarbiden und deren Mischungen bestehen, insbesondere aus Al₂O₃, MgO, ZrO₂, SiO₂, Cordierit, SiC, WC, B₄C, W, Au, Pt.

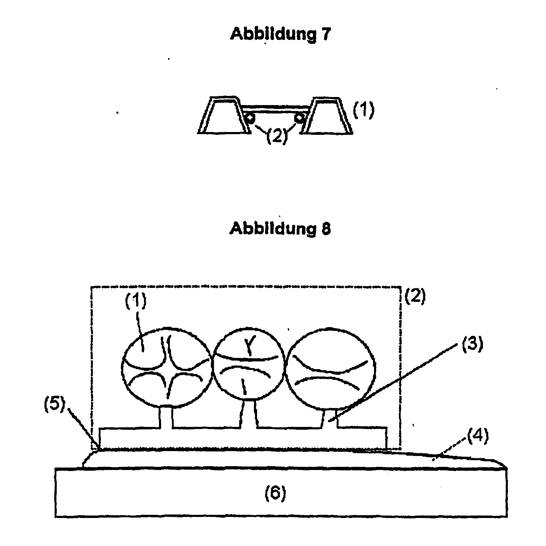

Die Abbildungen 6 und 7 zeigen weitere Ausführungsbeispiele für Gruppe I.

Die Abbildung 6 zeigt die Lagerung einer Brücke (1) auf Stäbchen (2), die flexibel innerhalb einer sogenannten Brennwatte (3) gelagert sind. Beim Sintervorgang können sich die Stäbchen (2) selbstständig in Richtung des Schrumpfes bewegen, ohne dass sie kippen oder die Brücke (1) deformieren.

Die Abbildung 7 zeigt eine andere Ausführungsform. Hierbei wird die prothetische Arbeit (1) auf eine rollenartige Konstruktion (2) gelegt, wobei sich die Abstände zwischen den Rollen im Laufe des Brennprozesses selbständig anpassen. Die Rollen werden auf geeigneten Aufhängungen bzw. Stützen, beispielsweise in T-oder U-Form, gelagert.

Bei kleinen keramischen Formgegenständen reichen einzelne oder einige wenige Träger und/oder Stützen aus. Bei großen Formgegenständen werden mehrere bis sehr viele Träger und/oder Stützen benötigt, die gegebenenfalls so gelagert sind, dass sich ihre Auflagepunkte der Form des zu sinternden Formgegenstandes anpassen können

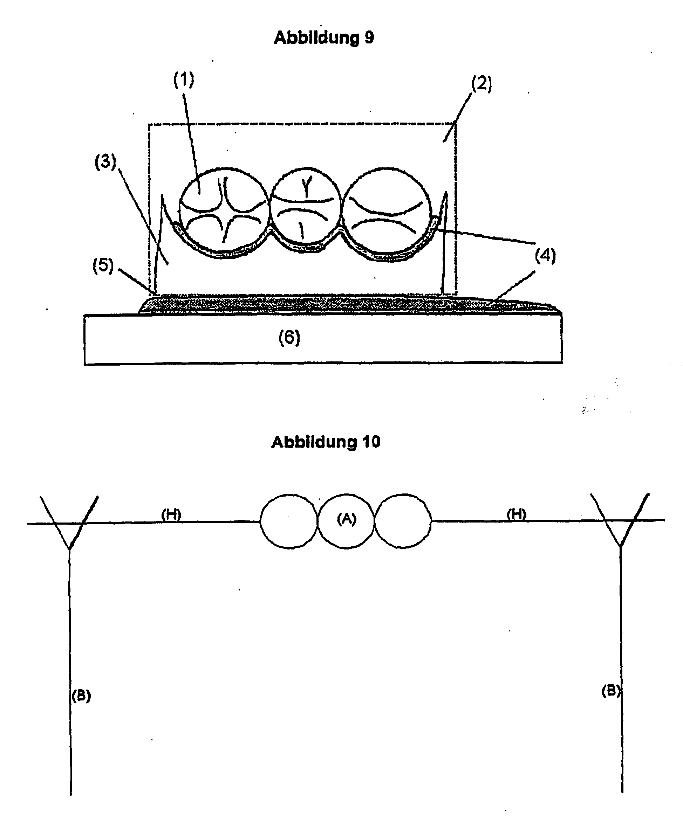

Mögliche Ausführungsformen zur Gruppe II der erfindungsgemäßen Verfahren sind nachfolgend wiedergegeben.
- Belassen der beim Fräsen des Werkstückes (1) notwendigen Haltestifte (3) nach dem Fräsvorgang, sodass diese als stabile Mehrpunktauflage auf einer ebenen Brennunterlage mit gleichem Schwindungsverhalten dienen. Die erfindungsgemäße Lagervorrichtung besteht in diesem Falle aus den Haltestegen (3) und einer planen Brennunterlage aus Material mit dem gleichen Schwindungsverhalten wie die prothetische Arbeit, vorzugsweise aus dem gleichen Material wie die prothetische Arbeit. Besonders bevorzugt wird während des Fräsvorgangs neben den Haltestiften (3) gleichzeitig eine plane Fläche (5) am Formkörper belassen, wobei der Rohling (2) entsprechend größer zu dimensionieren ist. Die Haltestifte (3) werden nach dem Sintem durchtrennt, um den gewünschten Formkörper zu erhalten. Die Vorrichtung für das erfindungsgemäße Verfahren wird beispielsweise über eine rieselfähige Schüttung (4) oder geeignete Träger und/oder Stützen auf eine feuerfeste Brennunterlage (6) gestellt. Die Abbildung 8 soll diese Ausführungsform genauer erläutern.
- Durchtrennen der Haltestifte noch vor dem Sintern, Aufbringen des Rest des ursprünglichen Rohlings (2), der nach dem Fräsen einer Negativform (3) der prothetischen Arbeit entspricht, auf eine plane Brennunterlage (5) über trennend wirkendem Pulver (4), Beschichten der Innenseite der Negativform (3) gleichfalls mit trennend wirkenden Pulver (4) sowie Auflegen der zu brennenden prothetischen Arbeit (1). Der Rohlingsrest (3) dient zusammen mit dem trennend wirkenden Pulver (4) als erfindungsgemäße Lagervorrichtung (Abbildung 9). Die Vorrichtung für das erfindungsgemäße Verfahren wird beispielsweise über eine rieselfähige Schüttung (4) oder geeignete Träger und/oder Stützen auf eine feuerfeste Brennunterlage (6) gestellt. Die Ausbildung von Sinterhälsen innerhalb der Schüttung aus trennend wirkendem Pulver findet überraschenderweise nicht statt.

Als trennend wirkende Pulver können alle refraktären Metalle, Metalloxide, Metallcarbide und deren Mischungen verwendet werden, insbesondere Al₂O₃, MgO, ZrO₂, SiO₂, Cordierit, SiC, WC, B₄C.

Die Abbildung 10 zeigt die Lagerung des Brenngutes (A) auf zwei Y-förmigen Trägern (B). Hierbei sind am Brenngut (A) zwei Haltestifte (H) befestigt, die entweder während des Formgebungsverfahrens erzeugt werden oder nach dem Formgebungsverfahren an das Brenngut angesetzt werden können. Die Haltestifte bestehen vorzugsweise aus dem gleichen Material wie das Brenngut, besonders bevorzugt sind sie aus dem gleichen Rohling gefertigt. Je nach Ausführungsform (unterschiedliches oder gleiches Material) ist diese Art der Lagerung der Gruppe I oder II zuzuordnen. Grundsätzlich kommen auch gemischte Ausführungsformen in Betracht, die gleichzeitig den verschiedenen Gruppen zuzuordnen sind.

## Patentansprüche

1. Verfahren zum dimensionstreuen Sintern von keramischen Formgegenständen, wobei das Brenngut während des Sinterns auf nicht mit Metall beschichteten, beweglichen Trägern gelagert wird, welche sich an die während des Brennprozesses auftretenden Schwunddimensionen selbständig anpassen und die aus einem beliebigen Material bestehen können, welches gegenüber dem Brennprozeß inert ist und keine Haftung zu dem Brenngut ergibt und dieses nicht verunreinigt, wobei die Träger in Fundamenten gelagert sind oder über eine Aufhängung befestigt sind.

2. Verfahren nach Anspruch 1, wobei die Formgegenstände keramische dentale Prothesen sind.

3. Verfahren nach Anspruch 1, wobei die Träger als senkrecht stehende oder waagrecht liegende hohle oder massive Stäbchen ausgebildet sind und einen Querschnitt aufweisen, der eine minimale Berührungsfläche mit dem Brenngut gestattet.

4. Verfahren nach Anspruch 1, wobei die Träger eine Spitze aufweisen, die eine minimale Berührungsfläche mit dem Brenngut gestattet, und hohl oder massiv sind.

5. Verfahren zum dimensionstreuen Sintern von keramischen Formgegensttänden, wobei das Brenngut während des Sinterns auf nicht mit Metall beschichteten Trägern gelagert wird, welche sich an die während des Brennprozesses auftretenden Schwunddimensionen selbständig anpassen, wobei die Lagerung des Brennguts auf Trägern erfolgt, die die gleichen physikalischen Eigenschaften aufweisen wie das Brenngut selbst, wobei Träger und Brenngut aus dem selben Rohling gefertigt sind und wobei das Brenngut über Haltestege, die nach dem Sintern durchtrennt werden, mit einer planen Fläche verbunden ist.

6. Verfahren zum dimensionstreuen Sintern von keramischen Formgegenständen, wobei das Brenngut während des Sinterns auf nicht mit Metall beschichteten Trägern gelagert wird, welche sich an die während des Brennprozesses auftretenden Schwunddimensionen selbständig anpassen, wobei die Lagerung des Brennguts auf Trägern erfolgt, die die gleichen physikalischen Eigenschaften aufweisen wie das Brenngut selbst, wobei Träger und Brenngut aus dem selben Rohling gefertigt sind und wobei das Brenngut in der durch den Fräsvorgang aus dem Rohling erhaltenen Negativform auf einer rieselfähigen Schüttung oder auf geeigneten Trägern und/oder Stützen gelagert wird.

7. verfahren nach Anspruch 5 oder 6, wobei die Formgegenstände keramische dentale Prothesen sind.

8. Verfahren nach Anspruch 5 oder 6, wobei der Rohling Aluminiumoxid, Zirkonoxid oder Mischoxide von beiden enthält.

## Claims

1. Process for the dimensionally-true sintering of ceramic pre-shaped items, the firing material resting during the sintering on movable supports, not coated with metal, which adapt independently to the shrinkage dimensions which occur during the firing process and which can be composed of any material which is inert vis-à-vis the firing process and does not result in adhesion to the firing material and does not contaminate the latter, the supports being housed in bases or attached via a suspension means.

2. Process according to claim 1, the pre-shaped items being ceramic dental prostheses.

3. Process according to claim 1, the supports being developed as vertically standing or horizontally lying hollow or solid rods and having a cross-section which allows a minimal contact surface with the firing material.

4. Process according to claim 1, the supports having a tip which allows a minimal contact surface with the firing material, and being hollow or solid.

5. Process for the dimensionally-true sintering of ceramic pre-shaped items, the firing material resting during the sintering on supports, not coated with metal, which adapt independently to the shrinkage dimensions which occur during the firing process, the firing material resting on supports which have the same physical properties as the firing material itself, support and firing material being prepared from the same preform and the firing material being connected to a plane surface via supporting pins which are cut through after sintering.

6. Process for the dimensionally-true sintering of ceramic pre-shaped items, the firing material resting during the sintering on supports, not coated with metal, which adapt independently to the shrinkage dimensions which occur during the firing process, the firing material resting on supports which have the same physical properties as the firing material itself, support and firing material being prepared from the same preform and the firing material resting in the negative mould obtained from the preform through the milling process on a pourable fill material or on suitable supports and/or props.

7. Process according to claim 5 or 6, the pre-shaped items being ceramic dental prostheses.

8. Process according to claim 5 or 6, the preform containing aluminium oxide, zirconium oxide or mixed oxides of both,

## Revendications

1. Procédé de frittage, fidèle en dimensions, d'objets moulés en céramique, la matière à chauffer étant placée pendant le frittage sur des supports mobiles qui ne sont pas revêtus de métal, qui s'adaptent automatiquement aux dimensions de retrait se présentant pendant le processus de chauffage et qui peuvent consister en une matière quelconque qui est inerte vis-à-vis du processus de chauffage et qui ne présente pas d'adhérence vis-à-vis de la matière à chauffer et ne contamine pas celle-ci, les supports étant placés dans des bases ou étant fixés par l'intermédiaire d'une suspension.

2. Procédé suivant la revendication 1, selon lequel les objets moulés sont des prothèses dentaires en céramique.

3. Procédé suivant la revendication 1, selon lequel les supports sont réalisés sous forme de petites tiges creuses ou pleines disposées verticalement ou horizontalement et ont une section transversale qui autorise une surface de contact minimale avec la matière à chauffer.

4. Procédé suivant la revendication 1, selon lequel les supports présentent une pointe autorisant une surface de contact minimale avec la matière à chauffer et ils sont creux ou pleins.

5. Procédé de frittage, fidèle en dimensions, d'objets moulés en céramique, selon lequel la matière à chauffer est placée pendant le frittage sur des supports qui ne sont pas rerêtus de métal, qui s'adaptent automatiquement aux dimensions de retrait se présentant pendant le processus de chauffage, la matière à chauffer étant supportée par des supports qui possèdent les mêmes propriétés physiques que la matière à chauffer elle même, les supports et la matière à chauffer étant réalisés à partir de la même ébauche et la matière à chauffer étant reliée à une surface plane par l'intermédiaire de tiges de maintien qui sont séparées après le frittage.

6. Procédé de frittage, fidèle en dimensions, d'objets moulés en céramique, la matière à chauffer étant placée pendant le frittage sur des supports qui ne sont pas revêtus de métal, qui s'adaptent automatiquement aux dimensions de retrait se présentant pendant le processus de chauffage, la matière à chauffer étant supportée par des supports qui possèdent les mêmes propriétés physiques que la matière à chauffer elle-même, les supports et la matière à chauffer étant réalisés à partir de la même ébauche et la matière à chauffer étant placée, dans le moule négatif obtenu au moyen de l'opération de fraisage à partir de l'ébauche, sur une matière en vrac apte à l'écoulement ou sur des supports appropriés et/ou sur des appuis.

7. Procédé suivant la revendication 5 ou 6, selon lequel les objets moulés sont des prothèses dentaires en céramique.

8. Procédé suivant la revendication 5 ou 6, selon lequel l'ébauche contient de l'oxyde d'aluminium, de l'oxyde de zirconium ou des mélanges des deux oxydes.
